# EUROPÄISCHE PATENTSCHRIFT

(11) **EP 1 926 821 B1**
(45) Veröffentlichungstag und Bekanntmachung des Hinweises auf die Patenterteilung: **20.04.2011**
(21) Anmeldenummer: 06762851.1
(22) Anmeldetag: 27.07.2006
(51) Int. Cl.: C12P 7/02

(54) **Verfahren zur enantioselektiven enzymatischen Reduktion von Ketoverbindungen**
Method of enantioselective enzymatic reduction of keto compounds
Procédé de reduction enzymatique enantioselective de composes cétoniques

(30) Priorität: 23.09.2005 AT 15702005
(43) Veröffentlichungstag der Anmeldung: 04.06.2008
(73) Patentinhaber: IEP GmbH, 65203 Wiesbaden (DE)
(72) Erfinder: GUPTA, Antje, 65207 Wiesbaden-Medenbach (DE); TSCHENTSCHER, Anke, 65347 Eltville-Hattenheim (DE); BOBKOVA, Maria, 65510 Idstein (DE)
(74) Vertreter: Schwarz, Albin
(86) Internationale Anmeldenummer: PCT/EP2006/007425
(87) Internationale Veröffentlichungsnummer: WO 2007/036257

(56) Entgegenhaltungen:
- WO-A-03/078615
- WO-A-2005/108593
- WO-A2-2004/111083
- US-A- 5 763 236
- HUMMEL WERNER: "Large-scale applications of NAD(P)-dependent oxidoreductases: Recent developments" TRENDS IN BIOTECHNOLOGY, ELSEVIER PUBLICATIONS, CAMBRIDGE, GB, Bd. 17, Nr. 12, Dezember 1999 (1999-12), Seiten 487-492, XP002237302 ISSN: 0167-7799
- SOMERS W A C ET AL: "Enantioselective oxidation of secondary alcohols at a quinohaemoprotein alcohol dehydrogenase electrode" APPLIED BIOCHEMISTRY AND BIOTECHNOLOGY - PART A ENZYME ENGINEERING AND BIOTECHNOLOGY 1998 UNITED STATES, Bd. 75, Nr. 2-3, 1998, Seiten 151-162, XP008070470 ISSN: 0273-2289

## Beschreibung

Die vorliegende Erfindung betrifft ein Verfahren zur enantioselektiven enzymatischen Reduktion von Ketoverbindungen mit Carbonylreduktasen.

Carbonylreduktasen (weitere Bezeichnungen: Alkoholdehydrogenasen, Oxidoreduktasen) sind als Katalysatoren zur Reduktion von Carbonylverbindungen bzw. zur Oxidation von sekundären Alkoholen bekannt. Diese Enzyme benötigen ein Co-Enzym, z.B. NAD(P)H. Die Reduktion von Ketonen mit der aus Lactobacillus kefir gewonnenen Carbonylreduktase und dem Co-Enzym NADPH ist z.B. aus der US 5,342,767 bekannt. Es gelingt mit diesen Enzymen, Ketoverbindungen zu optisch aktiven Hydroxyverbindungen zu reduzieren. Ein weiteres Verfahren ist beispielsweise aus der WO 03/078615 bekannt.

Optisch aktive Hydroxyverbindungen sind wertvolle chirale Bausteine mit breiter Anwendung für die Synthese von pharmakologisch wirksamen Verbindungen, aromatischen Substanzen, Pheromonen, Agrochemikalien und Enzyminhibitoren. Dabei ist insbesondere in der pharmazeutischen Industrie ein steigender Bedarf an chiralen Verbindungen und somit chiralen Synthesetechnologien zu verzeichnen, da racemische Verbindungen in Zukunft kaum noch als Arzneimittel Verwendung finden werden.

Die asymmetrische Reduktion prochiraler Ketoverbindungen ist ein Sektor der stereoselektiven Katalyse, in dem die Biokatalyse eine leistungsfähige Konkurrenztechnologie zur chemischen Katalyse darstellt. Die chemische asymmetrische Hydrierung erfordert den Einsatz hochgiftiger und umweltbelastender Schwermetallkatalysatoren, extremer und somit energieintensiver Reaktionsbedingungen sowie große Mengen organischer Lösungsmittel. Ferner sind diese Verfahren häufig gekennzeichnet durch Nebenreaktionen und unzureichende Enantiomerenüberschüsse.

Reduktionen prochiraler Ketoverbindungen zu Hydroxyverbindungen und umgekehrt kommen in der Natur in zahlreichen biochemischen Pathways, sowohl im Primärstoffwechsel als auch im Sekundärstoffwechsel, in jedem Organismus vor und werden von unterschiedlichen Typen von sekundären Alkoholdehydrogenasen und Oxidoreduktasen katalysiert. Diese Enzyme sind in der Regel cofaktorabhängig.

Die prinzipielle Machbarkeit der Nutzung von Biokatalysatoren zu Reduktion von prochiralen Ketoverbindungen zu chiralen Hydroxyverbindungen wurde in der Vergangenheit wiederholt anhand von Modellsystemen demonstriert, wobei sowohl mit isolierten Oxidoreduktasen als auch mit unterschiedlichen Ganzzellbiotransformationssystemen gearbeitet wurde. Der biokatalytische Ansatz ist hinsichtlich der milden Reaktionsbedingungen, fehlender Nebenprodukte-und oft wesentlich besseren erreichbaren Enantiomerenüberschüssen vorteilhaft. Dabei ist die Verwendung isolierter Enzyme gegenüber Verfahren mit ganzen Zellen hinsichtlich des erreichbaren Enantiomerenüberschuss, der Enstehung von Abbau-und Nebenprodukten als auch hinsichtlich der Produktisolation im Vorteil. Des weiteren ist die Verwendung von Ganzzellprozessen nicht jedem Chemieunternehmen möglich, da dafür spezielle Ausrüstung und Know how erforderlich sind,

Jüngst konnte gezeigt werden, daß die Verwendung isolierter Oxidoreduktasen in wäßrig/organischen Zweiphasen-Systemen mit organischen Lösungsmitteln äußerst effizient und auch in hohen Konzentrationen (> 5 %) möglich ist. Bei den beschriebenen Systemen bildet dabei die zu reduzierende, meist schlecht wasserlösliche Ketoverbindung zusammen mit dem organischen Lösungsmittel die organische Phase. Teilweise kann auch auf das organische Lösungsmittel selbst verzichtet werden, dann wird die organische Phase von der zu reduzierenden Ketoverbindung gebildet (DE10119274, DE10327454.4, DE 103 37 401.9, DE 103 00 335.5). Die Coenzymregenerierung wird dabei durch die gleichzeitige Oxidation sekundärer Alkohole realisiert, wobei in den meisten Fällen das preiswerte wassermischbare 2-Propanol verwendet wird.

Beispiele für geeignete R-und S-spezifische Oxidoreduktasen und Dehydrogenasen hoher Enantioselektivität sind:
*Carbonylreduktase aus Candida parapsilosis* (CPCR) (US 5,523,223 und US 5,763,236,(Enzyme Microb Technol. 1993 Nov; 15(11):950-8))
   oder *Pichia capsulata ADH* (DE10327454.4);
*Carbonylreduktase aus Rhodococcus erythropolis* (RECR) (US 5,523,223), *Norcardia fusca* (Biosci. Biotechnol. Biochem.,63 (10) (1999), Seiten 1721-1729), (Appl Microbiol Biotechnol. 2003 Sep;62(4):380-6. Epub 2003 Apr 26), und *Rhodococcus ruber* (J Org Chem. 2003 Jan 24;68(2):402-6.);
   und
R-spezifische sekundären Alkoholdehydrogenasen aus Organismen der Gattung *Lactobacillus* (Lactobacillus kefir (US5200335), Lactobacillus brevis (DE 19610984 A1) (Acta Crystallogr D Biol Crystallogr. 2000 Dec;56 Pt 12:1696-8), Lactobacillus minor (DE10119274) oder *Pseudomonas* (US 05385833)(Appl Microbiol Biotechnol. 2002 Aug;59(4-5):483-7. Epub 2002 Jun 26.,J. Org. Chem. 1992, 57, 1532);

Die WO 2005/108593 A beschreibt ein Verfahren zur enantioselektiven enzymatischen Reduktion von 2-butanon zur entsprechenden chiralen Hydroxyverbindung, wobei die Ketoverbindung in einer flüssigen, zweiphasischen Mischung, enthaltend 2-Butanon, 4-Methyl-2-pentanol oder 2-Heptanol und Wasser, mit einer Oxidoreduktase aus Candida parapsilosis in Gegenwart von NAD als Cofaktor umgesetzt wird.

In der WO 03/078615 A werden Alkoholdehydrogenasen sowie ein enantioselcktives enzymatisches Reduktionsverfahren von Ketoverbindungen unter Verwendung dieser Enzyme beschrieben, bei dem die Reduktion mit einer Oxidoreduktase in Gegenwart von z.B. NADH durchgeführt wird. Als Cosubstrate werden sekundäre Alkohole wie Isopropanol oder 4-Methyl-2-propanol eingesetzt.

Bei den Verfahren des Standes der Technik besteht der Bedarf, die Coenzymregenerierung zu verbessern bzw. zu vereinfachen. Die meisten Alkoholdehydrogenasen und Oxidoreduktasen werden bei Propanolkonzeatration von >15 % Vol.-% schnell inaktiviert, was dazu führt, daß dieses in Batchverfahren nicht in beliebigem Überschuss zur Keroverbindung einsetzbar ist, wodurch bei gleicher Konzentration an Ketoverbindung bei Substraten mit ungünstiger Gleichgewichtslage nur unbefriedigende Umsätze erreicht werden können.

Die Erfindung stellt sich die Aufgabe, diesen Nachteil zu beseitigen.

Das erfindungsgemäße Verfahren zur enantioselektiven enzymatischen Reduktion von
a) Ketoverbindungen der allgemeinen Formel I

   R₁-C(O)-R₂ (I)

   in der R1 für einen der Reste
   1) -(C₃-C₂₀)-Alkyl, worin Alkyl geradkettig oder verzweigtkettig ist,
   2) -(C₂-C₂₀)-Alkenyl, worin Alkenyl geradkettig oder verzweigtkettig ist und gegebenenfalls bis zu vier Doppelbindungen enthält,
   3) -(C₂-C₂₀)-Alkinyl, worin Alkinyl geradkettig oder verzweigtkettig ist und gegebenenfalls bis zu vier Dreifachbindungerl enthält,
   4) -(C₆-C₁₄)-Aryl.
   5) -(C₁-C₈)-Alkyl-(C₆-C₁₄)-Aryl,
   6) -(C₅-C₁₄)-Heterocyclus, der unsubstituiert oder ein-, zwei- oder dreifach durch -OH, Halogen, -NO₂ und/oder -NH₂ substituiert ist, oder
   7) -(C₃-C₇)-Cycloalkyl,
      steht, wobei die oben unter 1) bis 7) genannten Reste unsubstituiert sind oder unabhängig voneinander ein-, zwei- oder dreifach durch -OH, Halogen, -NO₂ und/oder -NH₂ substituiert sind,
      und R₂ für einen der Reste
   8) -(C₁-C₆)-Alkyl, worin Alkyl geradkettig oder verzweigtkettig ist,
   9) -(C₂-C₆)-Alkenyl, worin Alkenyl geradkettig oder verzweigtkettig ist und gegebenenfalls bis zu drei Doppelbindungen enthält,
   10) -(C₂-C₆)-Alkinyl, worin Alkinyl geradkettig oder verzweigtkettig ist und gegebenenfalls zwei Dreifachbindungen enthält, oder
   11) -(C₁-C₁₀)-Alkyl-C(O)-O-(C₁-C₆)-Alkyl, worin Alkyl gerade oder verzweigtkettig ist und unsubstituiert ist oder ein-, zwei- oder dreifach durch -OH, Halogen, -NO₂ und/oder -NH₂ substituiert ist,
   steht, wobei die oben unter 8) bis 11) genannten Reste unsubstituiert sind oder unabhängig voneinander ein-, zwei- oder dreifach durch *-*OH*,* Halogen*, -*NO₂ und/oder -NH₂ substituiert sind*,*
b) der Ketoverbindugen Ethyl-4-chloracetoacetat*,* Methylacetoacetat, 4-Hydroxy-2-butanon, Ethylpyruvat. Ethylphenylglyoxylat, 1,4-Dichlor-2-butanon, Ethyl-4-bromoacetoacetat*,* 1,1-Dichloraceton, 1,1,3-Trichloraceton, 1,1,1-Trifluoraceton, 1*-*Chloraceton oder 2,5-Hexandion
dadurch gekennzeichnet, daß
eine flüssige, zweiphasige Mischung, welche
(a) mindestens 5 Gew./Vol.-% eine Verbindung der Formel (I),
(b) mindestens 10 Vol.-% 4-Methyl-2-pentanol, 5-Methyl-2-hexanol und/oder 2-Heptanol und
(c) Wasser
enthält, mit einer Oxidoreduktase in Gegenwart von NAD(P)H als Co-Faktor behandelt wird, um für Fall (a) eine chirale Hydroxyverbindung der allgemeinen Formel II

R₁-CH(OM-R₂ (II)

zu bilden, in welcherR₁ und R₂ die oben angegebene Bedeutung haben, und im Fall (b) eine entsprechende chirale Hydroxyverbindung zu bilden*.*

Die Erfindung beruht auf der Erkenntnis, daß Verfahren, welche die hochexprimierierten, isolierten Alkoholdehydrogenasen und Oxidoreduktasen verwenden, durch Einsatz des nicht mit Wasser mischbaren 4-Methyl-2-pentanols, 5-Methyl-2-hexanols und/oder 2-Heptanols zur Coenzymregerienerung von NAD(P)H entscheidend verbessert bzw. vereinfacht werden können.

Bevorzugte Varianten des erfindungsgemäßen Vorfahrens sind dadurch gekennzeichnet, daß die flüssige, zweiphasige Mischung bei Anwendung einer Oxidoreduktase mikrobiellen Ursprungs mindestens 40 Vol.-%, insbesondere zwischen 40 und 80 Vol.%, 4-Methyl-2-pentanol, 5-Methyl-2-hexanol und/oder 2-Heptanol, bezogen auf das Gesamtvolumen des Reaktionsansatzes, enthält.

Im erfindungsgemäßen Verfahren wird die Reduktion der Ketoverbindung somit in einem ZweiPhasensystem durchgeführt, bestehend aus einer wäßrigen Phase, den Cofaktor NADH oder NADPH und die Oxidoreduktase enthaltend, und einer organischen Phase, gebildet vom Cosubstrat 4-Methyl-2-pentanol und von der größtenteils darin gelösten Ketoverbindung.

Die Coenzymregeneration von NAD(P)H erfolgt dabei durch Oxidation des Cosubstrates 4-Methyl-2-pentanol, 5-Methyl-2-hexanol und/oder 2-Heptanol, das gleichzeitig als Lösungs-und Extraktionsmittel insbesondere für schwer wasserlösliche Ketoverbindungen dient

Durch den Einsatz von 4-Methyl-2-pentanol, 5-Methyl-2-hexanol und/oder 2-Heptanol als Lösungsmittel und Cosubstrat können auch für Substrate mit ungünstiger Gleichgewichtslage gute Umsätze (>90%), hohe Konzentrationen sowie wesentlich kürzere Reaktionszeiten realisiert werden.

Besonders vorteilhaft ist das beschriebene Verfahren auch für die Reduktion von Ketonen mit niedrigen Siedepunkten, wie z.B. 1,1,1-Trifluoraceton, und solcher, bei denen die Siedepunkte der resultierenden chiralen Alkohole, wie im Falle von 1,1,1-Trifluorpropan-2-ol unter dem von Wasser liegt. In diesen Fällen ist oft die Trennung von Hydroxyverbindungen, Aceton, 2-Propanol und Wasser mittels Destillation erschwert.

Desweiteren haben sich die erfindungsgemäß eingesetzten Alkohole als stabilisierend auf viele eingesetzte Oxidoreduktasen erwiesen, was allgemein zu einem verringertem Enzymverbrauch verglichen mit anderen wäßrig-organischen Zwei-Phasensystemen führt.

Die Coenzymregenerierung kann dabei substratgekoppelt, (d.h. ein Enzym zur Reduktion des Ketosubstrates und zur Oxidation des 4-Methyl-2-pentanol) oder enzymgekoppelt erfolgen. Beim enzymgekoppelten Ansatz erfolgt die Regeneration des Cofaktors NADH oder NADPH mit Hilfe einer zweiten hochexprimierten isolierten sekundären Alkoholdehydrogenase.

Mit dieser Methode werden ttn's (total turn over number, mol Produkt gebildet pro mol Cofaktor) erreicht die im Bereich von10³ -10⁶ liegen. Die realisierbaren Substratkonzentrationen liegen dabei größtenteils weit über 5 % (Volumenprozent).

Die Konzentration des Cosubstrates liegt dabei im Bereich von 10 % bis 90 % Vol.-% des Reaktionsgemisches, bevorzugt zwischen 40 und 80 Vol.%.

Der Enzymverbrauch der Oxidoreduktase liegt im Bereich von 10 000 - 10 Mio U/kg (nach oben offen) umzusetzende Ketoverbindung. Der Enzymeinheit 1 U entspricht dabei der Enzymmenge die benötigt wird um 1 µmol der Verbindung der Formel 1 je Minute (min) umzusetzen.

Unter dem Begriff "NADH" wird reduziertes Nicotinamid-adenin-dinucleotid verstanden. Unter dem Begriff "NAD" wird Nicotinamid-adenin-dinucleotid verstanden. Unter dem Begriff "NADPH" wird reduziertes Nicotinamid-adenin-dinucleotid-phosphat verstanden. Unter dem Begriff "NADP" wird Nicotinamid-adenin-dinucleotid-phosphat verstanden.

Unter dem Begriff chirale "Hydroxyverbindung" werden beispielsweise Verbindungen der Formel II

R1-C(OH)-R2 (II)

verstanden, wobei R1 und R2 die Bedeutung wie in Formel I haben.

Unter dem Begriff Aryl werden aromatische Kohlenstoffreste verstanden mit 6 bis 14 Kohlenstoffatomen im Ring. -(C₆-C₁₄)- Arylreste sind beispielsweise Phenyl, Naphthyl, zum Beispiel 1-Naphthyl, 2-Naphthyl, Biphenylyl, zum Beispiel 2-Biphenylyl, 3-Biphenyl und 4-Biphenylyl, Anthryl oder Fluorenyl. Diphenylylreste, Naphthylreste und insbesondere Phenylreste sind bevorzugte Arylreste. Unter dem Begriff "Halogen" wird ein Element aus der Reihe Fluor, Chlor, Brom oder Jod verstanden. Unter dem Begriff "-(C₁-C₂₀)-Alkyl wird ein Kohlenwasserstoffrest verstanden, dessen Kohlenstoffkette geradkettig oder verzweigt ist und 1 bis 20 Kohlenstoffatome enthält beispielsweise Methyl, Ethyl, Propyl, Isopropyl, Butyl, tertiär-Butyl, Pentyl, Hexyl, Heptyl, Octyl, Nonenyl oder Decanyl. Unter dem Begriff "-(C₃-C₇)-Cycloalkyl" werden cyclische Kohlenwasserstoffreste verstanden wie Cyclopropyl. Cylobutyl, Cyclopentyl, Cyclohexyl oder Cycloheptyl. Der Begriff "-(C₅-C₁₄)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, 0 und S. Beispiele für die Begriffe -(C₅-C₁₄)-Heterocyclus sind Reste, die sich von Pyrrol, Furan, Thiophen, Imidazol, Pyrazol, Oxazol, Isoxazol, Thiazol, Isothiazol, Tetrazol, 1,2,3,5-oxathiazol-2-Oxide, Triazolone, Oxadiazolone, Isoxazolone, Oxadiazolidindione, Triazole, welche durch F, -CN. -CF₃ oder -C(O)-O-(C₁-C₄)-Alkyl substituert sind, 3-Hydroxypyrro-2,4-dione. 5-Oxo-1,2,4-Thiadiazole, Pyridin, Pyrazin, Pyrimidin, Indol, Isoindol, Indazol, Phthalazin, Chinolin, Isochinolin, Chinoxalin, Chinazolin, Cinnolin, - Carbolin und benz-anellierte, cyclopenta-, cyclohexa- oder cyclohepta-anellierte Derivate dieser Heterocyclen ableiten. Insbesondere bevorzugt sind die Reste 2- oder 3-Pyrrolyl, Phenylpyrrolyl wie 4- oder 5-Phenyl-2-pyrrolyl, 2-Furyl, 2-Thienyl, 4-Imidazolyl, Methyl-imidazolyl, zum Beispiel 1-Methyl-2-, -4- oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder -3-indoxyl, 4,5,6,7-Tetrahydro-2-indolyl, Cycichepta[b]-5-pyrrolyl, 2., 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzo-thienyl, 2-Benzoxazolyl oder Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Ketoverbindungen sind Ethyl-4-chloracetoacetat, Methylacetoacetat, Ethyl-8-chloro-6-oxooctansäure, Ethyl-3-oxovaleriat, 4-Hydroxy-2-butanon, Ethyl-2-oxovaleriat, Ethyl-2-oxo-4-phenylbuttersäure, Ethylpyruvat, Ethylphenylglyoxylat, 1-Phenyl-2-propanon, 2,3-Dichloracetophenon, Acetophenon, 2-Octanon, 3-Octanon, 2,5-Hexandion, 1,4-Dichlor-2-butanon, Phenacylchlorid, Ethyl-4-bromoacetoacetat, 1,1-Dichloraceton, 1,1,3-Trichloraceton, 1,1,1-Trifluoraceton und 1-Chloraceton.

Im erfindungsgemäßen Verfahren kann das Enzym entweder vollständig gereinigt, teilweise gereinigt oder in Zellen enthalten eingesetzt werden. Die eingesetzten Zellen können dabei nativ, permeabilisiert oder lysiert vorliegen.

Je kg umzusetzender Verbindung der Formel I werden 10 000 bis 10 Mio U Oxidoreduktase eingesetzt. (nach oben offen) Der Enzymeinheit 1 U entspricht dabei der Enzymmenge die benötigt wird um 1 µmol der Verbindung der Formel I je Minute (min) umzusetzen.

Zusätzlich zur Oxidoreduktase für die enantioselektive Ketoreduktion kann noch eine weitere Oxidoreduktase, bevorzugt eine sekundäre Alkoholdehydrogenase, zur Coenzymregenerierung enthalten sein. Geeignete sekundäre Alkoholdehydrogenasen sind beispielsweise die aus Thermoanaerobium brockii, Clostridium beijerinckii, Lactobacillus minor oder Lactobacillus brevis, Pichia capsulata, Candida parapsilosis, Rhodococcus erythropolis.

Die Alkohol-Dehydrogenase kann in dem erfindungsgemäßen Verfahren entweder vollständig gereinigt oder teilweise gereinigt eingesetzt werden oder es können ganze Zellen, die die Alkohol-Dehydrogenase enthalten, verwendet werden. Die eingesetzten Zellen können dabei nativ, permeabilisiert oder lysiert vorliegen.

Dem Wasser kann ein Puffer zugesetzt wer den, beispielsweise Kaliumphosphat-, Tris/HCl- oder Triethanolamin-Puffer mit einem pH-Wert von 5 bis 10, vorzugsweise ein pH-Wert von 6 bis 9. Der Puffer kann zusätzlich noch Ionen zur Stabilisierung oder Aktivierung beider Enzyme enthalten, beispielsweise Magnesiumionen zur Stabilisierung der Alkohol-Dehydrogenase aus Lactobaillus minor.

Das Substrat kann fest oder flüssig, wasserlöslich oder wasserunlöslich sein. Das Substrat kann ferner während der Reaktion vollständig oder auch unvollständig gelöst vorliegen. Dem Reaktionsansatz kann ein zusätzliches organisches Lösungsmittel enthalten. Die bevorzugten organischen Lösungsmittel sind beispielsweise Ethylacetat, tertiär-Butylmethylether, Diisopropylether, Heptan, Hexan oder Cyclohexan oder deren Gemische unterschiedlicher Zusammensetzung.

Die Konzentration des Cofaktors NAD(P)H bezogen auf die wässrige Phase beträgt von 0,001 mM bis 1 mM, insbesondere von 0,01 mM bis 0,1 mM.

Die Verbindungen der Formel I werden im erfindungsgemäßen Verfahren beispielsweise in einer Menge von 2% -50% (w/v) bezogen auf das Gesamtvolumen eingesetzt, bevorzugt von 10% bis 30% (w/v).

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Je nach Substrat und eingesetzter Verbindung der Formel I beträgt die Reaktionszeit von 1 Stunde bis 96 Stunden, insbesondere von 2 Stunden bis 24 Stunden.

Umgekehrt kann das erfindungsgemäße Verfahren auch für die enzymkatalysierte Oxidationsreaktion angewandt werden. Die Reaktionsbedingungen sind im wesentlichen dieselben wie im obengenannten Verfahren zur enantiospezifischen Reduktion der Ketoverbindung der Formel I. Im Verfahren wird jedoch statt einer enantioselektiven Reduktion der Ketoverbindung der Formel I, die ensprechende Hydroxyverbindung der Formel II zur entsprechenden Ketoverbindung oxydiert. Ferner werden im Verfahren anstelle von 4-Methyl-2-pentanol, 5-Methyl-2-hexanol bzw. 5-Methyl-3-heptanol die preiswerten korrespondierenden Ketone 4-Methyl-2-pentanon, 5-Methyl-2-hexanon bzw. 5 Methyl-3-heptanon zur Regenerierung von NAD(P) eingesetzt. Dabei wird bei Verwendung einer racemischen Hydroxyverbindung der Formel II in Verbindung mit einer enantioselektiven Oxidoreduktase die Ketoverbindung der Formel I und ein Enantiomer der racemischen Hydroxyverbindung der Formel II erhalten.

Das Verfahren kann aber auch zur Darstellung schwer zugänglicher Ketoverbindungen aus deren racemischen Alkoholen angewendet werden indem unselektive Oxidoreduktasen oder auch Gemische von enantioselektiven Oxidoreduktasen zum Einsatz kommen.

Die Erfindung wird nachfolgend an Hand von Beispielen noch näher erläutert.

### Beispiele

Die Reduktion der Verbindungen der Formel 1 wird durchgeführt in dem untenstehende Komponenten in ein Reaktionsgefäß überführt werden und bei guter Durchmischung bei Raumtemperatur inkubiert werden.

### 1. Synthese von (R)-Ethyl-4-chloro-3-hydroxybuttersäure mit NADH abhängigem Enzym aus Candida parapsilosis

| Komponente | Menge | Prozent | Konzentration |
|---|---|---|---|
| Puffer | | | |
| 100 mM Triethanolaminpuffer | 1 ml | | |
| pH = 7,5; 2mM ZnCl₂ 10 % Glycerin | | | |
| NAD [M = 663 g/mol] | 2 mg | 3 µmol | 0,3 mM |
| 4-Methyl-2-pentanol | 7 ml | | |
| Ethyl -4-chloracetoacetat | 2 ml | 20 % (v/v) | |
| (164g/mol, d= 1,2g/ml) | = 2,4 g | 14,6 mmol | |
| Enzym = S-ADH aus Candida parapsilosis | 1200 U | | |
| Volumen | 10 ml | | |
| System | biphasisch | | |
| Coenzymregenerierung | substratgekoppelt | | |
| Inkubationszeit | 24 h | | |
| Umsatz | >99 % | | |
| ee-Wert | >99 % | | |
| ttn NAD | 4866 | | |
| Enzymverbrauch | 500 U/g | | |

Nach Beendigung der Reaktion wird die wäßrige Phase, von der das Produkt enthaltenden organischen Phase abgetrennt und das Produkt (R) -Ethyl-4-chloro-3-hydroxybutyrate mitttels Destillation vom 4-Methyl-2-pentanol gereinigt. In dieser weise kann das (R) -Ethyl-4-chloro-3-hydroxybutyrate in hoher chemischer und optischer Reinheit gewonnen werden.

### 2. Synthese S,S- Butandiol mit NADH abhängigem Enzym aus Candida parapsilosis

| Komponente | Menge | Prozent | Konzentration |
|---|---|---|---|
| Puffer | | | |
| 100 mM Triethanolaminpuffer | 1 ml | | |
| pH = 7,5; 2mM ZnCl₂, 10 % Glycerin | | | |
| NAD [M = 663 g/mol] | 1 mg | 1,5 µmol | 0,15 mM |
| 4-Methyl-2-pentanol | 8 ml | | |
| 4-Hydroxy-2-butanon (M =88,12 gmol,) | 1 ml | 10 % (v/v) | |
| Enzym | | | |
| = S-ADH aus Candida parapsilosis | 1000 U | | |
| Volumen | 10 ml | | |
| System | biphasisch | | |
| Coenzymregenerierung | substratgekoppelt | | |
| Inkubationszeit | 24 h | | |
| Umsatz | > 90 | | |
| ee-Wert | > 99 % | | |
| ttn NAD | 6630 | | |
| Enzymverbrauch | 500 U/g | | |

Nach Beendigung der Reaktion wird die wäßrige Phase, von der das Produkt enthaltenden organischen Phase abgetrennt und das Produkt (S,S) Butandiol mittels Destillation vom 4-Methyl-2-pentanol gereinigt. In dieser Weise kann das (S,S) Butandiol in hoher chemischer und optischer Reinheit gewonnen werden

### 3. Synthese 2,5- S,S- Hexandiol mit NADH abhängigem Enzym aus Candida parapsilosis

| Komponente | Menge | Prozent | Konzentration |
|---|---|---|---|
| Puffer | | | |
| 100 mM Triethanolaminpuffer pH = | 100 ml | | |
| 7,5 | | | |
| NAD [M = 663 g/mol] | 100 mg | = 0,15 mmol | |
| 4-Methyl-2-pentanol | 800 ml | | |
| 2,5 Hexandion | 100 ml | 10 % (v/v) | |
| (114 g/mol, d= 1) | =0,87 mol | | |
| Enzym | | | |
| = S-ADH aus Candida parapsilosis | 36 000 U | | |
| Volumen | 1 l | | |
| System | biphasisch | | |
| Coenzymregenerierung | substratgekoppelt | | |
| Inkubationszeit | 24 h | | |
| Umsatz | 67 % | | |
| ee-Wert | >99,9 | | |
| ttn NAD | 5800 | | |
| Enzymverbrauch | 360 U/g | | |

Nach Beendigung der Reaktion wird die wäßrige Phase, von der das Produkt enthaltenden organischen Phase abgetrennt und das Produkt/Eduktgemisch 2,5- (S,S) Hexandiol/ 2,5-Hexandion mittels Destillation vom 4-Methyl-2-pentanol abgetrennt.
Das Produkt 2,5- (S,S) Hexandiol läßt sich in einer anschließenden Vakuumdestillation vom Edukt 2,5-Hexandion abtrennen und in einer chemische Reinheit >99 % gewinnen. Die Gesamtausbeute des Prozesses beträgt dabei beispielsweise 40-60 %.

### 4. Synthese (R)-2-Chlor-1-(3-chlorphenyl)ethan-1-ol mit NADH abhängigem Enzym aus Pichia capsulata

| Komponente | Menge | Prozent | Konzentration |
|---|---|---|---|
| 100 mM Triethanolaminpuffer | | | |
| pH = 7; 2mM ZnCl₂,10 % Glycerin | 1ml | | |
| NAD [M = 663 g/mol] | 0,5 mg | 0,75gmol | |
| 4-Methyl-2-pentanol | 8 ml | | |
| 2-Chlor-1-(3-chlorphenyl)ethan-1-on | 1 g | 5,2 mmol | |
| (M =189 gmol) | | | |
| Enzym | | | |
| = S-ADH aus Pichia capsulata | 1000 U | | |
| (DE 10327454.4) | | | |
| Volumen | 10 ml | | |
| System | biphasisch | | |
| Coenzymregenerierung | substratgekoppelt | | |
| Inkubationszeit | 24 h | | |
| Umsatz | > 99 % | | |
| ee-Wert | >99 % | | |
| ttn NAD | 6900 | | |
| Enzymverbrauch | 1000 U/g | | |

Nach Beendigung der Reaktion wird die wäßrige Phase, von der das Produkt enthaltenden organischen Phase abgetrennt und das Produkt 2-Chlor-1-(3-chlorphenyl)ethan-1-ol mittels Destillation vom 4-Methyl-2-pentanol abgetrennt.

### 5. Reduktion von 8- chloro-6-oxooktansäureethylester zu S-8-chloro-6-hydroxyoktansäureethylester mittels NADPH abhängiger Oxidoreduktase

| Komponente | Menge | Prozent | Konzentration |
|---|---|---|---|
| 100 mM Kaliumphosphatpuffer | 2 ml | | |
| pH = 8,5, 10 % Glycerin | | | |
| NADP [M = 765 g/mol] | 0,1 mg | 0,13 µmol | |
| 4-Methyl-2-pentanol | 5 ml | | |
| S-8-chloro-6-oxo-oktansäureethylester | 0,5 ml | 2,2 mmol | |
| 222.71 g/mol | | | |
| Enzym | | | |
| = Oxidoreduktase aus Lactobacillus | 240 U | | |
| reuteri | | | |
| DE 103 00 335.5 | | | |
| Enzym | 240 U | | |
| ADH aus Thermoanerobium brockii | | | |
| Volumen | 8 ml | | |
| System | biphasisch | | |
| Coenzymregenerierung | enzymgekoppelt | | |
| Inkubationszeit | 24 h | | |
| Umsatz | 90 % | | |
| ee-Wert | 97% | | |
| ttn NADP | 17 000 | | |
| Enzymverbrauch | 480 U/g | | |
| Lactobacillus reuteri | | | |
| Enzymverbrauch | 480 U/ | | |
| Thermoanerobium brockii | | | |

### 6. Reduktion von 3-oxovalerianssäure-methylester zu S-3-hydroxy-oxovalerianssäure-methylester Pichia capsulata

| Komponente | Menge | Prozent | Konzentration |
|---|---|---|---|
| Puffer | | | |
| 100 mM Triethanolaminpuffer pH = | 450 µl | | |
| 7,0 | | | |
| 10% Glycerin | | | |
| NAD [M = 663 g/mol] | 0,1 mg | | |
| 4-Methyl-2-pentanol | 450 µl | | |
| Methyl-3-oxövaleriate | 100 µl | | |
| Enzym | | | |
| = S-ADH aus Pichia capsulata | 50 U | | |
| (DE 10327454.4) | | | |
| Volumen | 1000 µl | | |
| System | biphasisch | | |
| Coenzymregenerierung | substratgekoppelt | | |
| Inkubationszeit | 24 h | | |
| Umsatz | 95% | | |
| ee-Wert | >99 % | | |
| ttn NAD | 5300 | | |
| Enzymverbrauch | 500 U/g | | |

## Patentansprüche

1. Verfahren zur enantioselektiven enzymatischen Reduktion
a) von Ketoverbindungen der allgemeinen Formel I
R₁-C(O)-R₂ (I)
in der R1 für einen der Reste
1) -(C₃-C₂₀)-Alkyl, worin Alkyl geradkettig oder verzweigtkettig ist,
2) -(C₂-C₂₀)-Alkenyl, worin Alkenyl geradkettig oder verzweigtkettig ist und gegebenenfalls bis zu vier Doppelbindungen enthält,
3) -(C₂-C₂₀)-Alkinyl, worin Alkinyl geradkettig oder verzweigtkettig ist und gegebenenfalls bis zu vier Dreifachbindungen enthält,
4) -(C₆-C₁₄)-Aryl,
5) -(C₁-C₈)-Alkyl-(C₆-C₁₄)-Aryl,
6) -(C₅-C₁₄)-Heterocyclus, der unsubstituiert oder ein-, zwei- oder dreifach durch -OH, Halogen, -NO₂ und/oder -NH₂ substituiert ist, oder
7) -(C₃-C₇)-Cycloalkyl,
steht, wobei die oben unter 1) bis 7) genannten Reste unsubstituiert sind oder unabhängig voneinander ein-, zwei- oder dreifach durch -OH, Halogen, -NO₂ und/oder -NH₂ substituiert sind,
und R₂ für einen der Reste
8) -(C₁-C₆)-Alkyl, worin Alkyl geradkettig oder verzweigtkettig ist,
9) -(C₂-C₆)-Alkenyl, worin Alkenyl geradkettig oder verzweigtkettig ist und gegebenenfalls bis zu drei Doppelbindungen enthält,
10) -(C₂-C₆)-Alkinyl, worin Alkinyl geradkettig oder verzweigtkettig ist und gegebenenfalls zwei Dreifachbindungen enthält, oder
11) -(C₁-C₁₀)-Alkyl--C(O)-O-(C₁-C₆)-Alkyl, worin Alkyl gerade oder verzweigtkettig ist und unsubstituiert ist oder ein-, zwei- oder dreifach durch -OH, Halogen, -NO₂ und/oder - NH₂
substituiert ist,
steht, wobei die oben unter 8) bis 11) genannten Reste unsubstituiert sind oder unabhängig voneinander ein-, zwei- oder dreifach durch -OH, Halogen, -NO₂ und/oder -NH₂ substituiert sind,
b) der Ketoverbindungen Ethyl-4-chloracetoacetat, Methylacetoacetat, 4-Hydroxy-2-butanon, Ethylpyruvat, Ethylphenylglyoxylat, 1,4-Dichlor-2-butanon, Methyl-4-Poromoacetoacetat, 1,1-Dich9oraceton, 1,1,3-Trichloraceton, 1,1,1-Trifluoraceton, 1-Chloraceton oder 2,5-Hexandion
**dadurch gekennzeichnet, daß**
eine flüssige, zweiphasige Mischung, welche
(a) mindestens 5 Gew./Vol.-% einer der obengenannten ketoverbindungen,
(b) mindestens 10 Vol.-% 4-Methyl-2-pentanol, 5-Methyl-2-hexanol und/oder 2-Heptanol und
(c) Wasser
enthält, mit einer Oxidoreduktase in Gegenwart von NAD(P)H als Co-Faktor behandelt wird, um für Fall (a) eine chirale Hydroxyverbindung der allgemeinen Formel II
R₁-CH(OH)-R₂ (II)
zu bilden, in welcher R₁ und R₂ die oben angegebene Bedeutung haben, und im Fall (b) eine entsprechende chirale, Hydroxyverbindung zu bilden.

2. Verfahren nach Anspruch 1, **dadurch gekennzeichnet, daß** die Oxidoreduktase mikrobiellen Ursprungs ist.

3. Verfahren nach Anspruch 1, **dadurch gekennzeichnet, daß** die Oxidoreduktase aus Bakterien der Gruppe Lactobacillales oder aus Hefen stammt.

4. Verfahren nach Anspruch 3, **dadurch gekennzeichnet, daß** die Oxidoreduktase aus Bakterien der Gattung *Lactobacillus* oder aus Hefen der Gattungen *Pichia, Candida, Pachysolen, Debaromyces* oder *Issatschenkia* stammt.

5. Verfahren nach einem der Ansprüche 1 bis 4, **dadurch gekennzeichnet, daß** die flüssige, zweiphasige Mischung bei Anwendung einer Oxidoreduktase mikrobiellen Ursprungs mindestens 40 Vol.-% 4.-Methyl-2-pentanol, 5-Methyl-2-hexanol und/oder 2-Heptanol enthält.

6. Verfahren nach Anspruch 5, **dadurch gekennzeichnet, daß** die flüssige, zweiphasige Mischung zwischen 40 und 80 Vol.-% 4-Methyl-2-pentanol, 5-Methyl-2-hexanol und/oder 2-Heptanol enthält.

7. Verfahren nach einem der Ansprüche 1 bis 6, **dadurch gekennzeichnet, daß** die flüssige, zweiphasige Mischung eine der obengenannten Ketoverbindungen zwischen 2 und 50 Gew./Vol.-% enthält.

8. Verfahren nach Anspruch 7, **dadurch gekennzeichnet, daß** die flüssige, zweiphasige Mischung eine der obengenannten Ketoverbindungen zwischen 10 und 50 Gew./Vol.-% enthält

## Claims

1. A process for the enantioselective enzymatic reduction
a) of keto compounds of general Formula I
R₁-C(O)-R₂ (I)
wherein R1 stands for one of the moieties
1) -(C₃-C₂₀)-alkyl, wherein alkyl is linear-chain or branched,
2) -(C₂-C₂₀)-alkenyl, wherein alkenyl is linear-chain or branched and optionally contains up to four double bonds,
3) -(C₂-C₂₀)-alkynyl , wherein alkynyl is linear-chain or branched and optionally contains up to four triple bonds,
4) -(C₆-C₁₄)-aryl,
5) "(C₁-C₈)-alkyl-(C₆-C₁₄)-alkyl,
6) -(C₅-C₁₄)-heterocycle which is unsubstituted or substituted one, two or three times by -OH, halogen, NO₂ and/or -NH₂, or
7) -(C₃-C₇)-cycloalkyl,
wherein the moieties mentioned above under 1) to 7) are unsubstituted or substituted one, two or three times, independently of each other, by -OH, halogen, -NO₂ and/or -NH₂,
and R₂ stands for one of the moieties
8) -(C₁-C₆)-alkyl, wherein alkyl is linear-chain or branched,
9) -(C₂-C₆)-alkenyl, wherein alkenyl is linear-chain or branched and optionally contains up to three double bonds,
10) -(C₂-C₆)-alkenyl, wherein alkynyl is linear-chain or branched and optionally contains two triple bonds, or
11) -(C₁-C₁₀)-alkyl-C(O)-O-(C₁-C₆)-alkyl, wherein alkyl is linear or branched and is unsubstituted or substituted one, two or three times by -OH, halogen, -NO₂ aud/or -NH₂, wherein the moieties mentioned above under 8) to 11) are unsubstituted or substituted one, two or three times, independently of each other, by -OH, halogen, -NO₂ and/or -NH₂,
b) of the keto compounds ethyl-4-chloroacetoacetate, methylacetoacetate, 4-hydroxy-2-butanone, ethylpyruvate, ethylphenylglyoxylate, 1,4-dichloro-2-butanone, ethyl-4-bromoacetoacetate, 1,1-dichloroacetone, 1,1 ,3-trichloroacetone, 1,1,1-trifluoroacetone, 1-chloroacetone or 2,5-hexanedione
**characterized in that**
a liquid, two-phase mixture containing
(*a*) at least 5% by weight/by volume of one of the above-mentioned keto compounds,
*(b)* at least 10% by volume of 4-methyl-2-penranol, 5-methyl-2-hexanol and/or 2-heptanol and
(*c*) water,
is treated with an oxidoreductase in the presence of NAD(P)H as a cofactor in order to form in case of (a) a chiral hydroxy compound of general Formula II
R₁-CH(OH)-R₂ (II)
wherein R₁ and R₂ have the above-indicated meanings, and to form in case of (b) a corresponding chiral hydroxy compound.

2. The process according to claim 1, **characterized in that** the oxidoreductase is of microbial origin.

3. The process according to claim 1, **characterized in that** the oxidoreductase originates from bacteria of the group of Lactobacillales or from yeasts.

4. The process according to claim 3, **characterized in that** the oxidoreductase originates from bacteria of the genus *Lactobacillus* or from yeasts of the genera *Pichia, Candida, Pachysolen, Debaromyces* or *Issatschenkia.*

5. The process according to any of claims 1 to 4, **characterized in that** the liquid, two-phase mixture contains at least 40% by volume of 4-methyl-2-pentanol, 5-methyl-2-hexanol and/or 2-heptanol if an oxidoreductase of microbial origin is used.

6. The process according to claim 5, **characterized in that** the liquid, two-phase mixture contains between 40 and 80% by volume of 4-methyl-2-pentanol, 5-methyl-2-hexanol and/or 2-heptanol.

7. The process according to any of claims 1 to 6, **characterized in that** the liquid, two-phase mixture contains one of the above-mentioned keto compounds in an amount of between 2 and 50% by weight/by volume.

8. The process according to claim 7, **characterized in that** the liquid, two-phase mixture contains one of the above-mentioned keto compounds in an amount of between 10 and 50% by weight/by volume.

## Revendications

1. Procédé de réduction enzymatique énantiosélective de
a) composés céto de formule générale I
R₁C(O)-R₂ (I)
dans laquelle R₁ représente l'un des groupes suivants :
1) un alkyle en C₃ à C₂₀, l'alkyle étant à chaîne linéaire ou ramifiée,
2) un alcényle en C₂ à C₂₀, l'alcényle étant à chaîne linéaire ou ramifiée et contenant éventuellement jusque quatre doubles liaisons,
3) un alcinyle en C₂ à C₂₀, l'alcinyle étant à chaîne linéaire ou ramifiée et contenant éventuellement jusque quatre triples liaisons,
4) un aryle en C₆ à C₁₄,
5) un (alkyle en C₁ à C₈) (aryle en C₆ à C₁₄),
6) un hétérocycle en C₅ à C₁₄ qui est non substitué ou substitué une fois, deux fois ou trois fois avec -OH, un halogène, -NO₂ et/ou -NH₂ ou
7) un cyclo(alkyle en C₃ à C₇),
les groupes mentionnés aux points 1) à 7) étant non substitués ou étant substitués indépendamment l'un de l'autre une, deux ou trois fois avec -OH, un halogène, -NO₂ et/ou -NH₂,
R₂ représentant un des groupes suivants :
8) un alkyle en C₁ à C₆, l'alkyle étant à chaîne linéaire ou ramifiée,
9) un alcényle en C₂ à C₆, l'alcényle étant à chaîne linéaire ou ramifiée et contenant éventuellement jusque trois doubles liaisons,
10) un alcinyle en C₂ à C₆, l'alcinyle étant à chaîne linéaire ou ramifiée et contenant éventuellement deux triples liaisons ou
11) un (alkyle en C₁ à C₁₀)-C-(O)-O-(alkyle en C₁ à C₆), l'alkyle étant à chaîne linéaire ou ramifiée et étant non substitué ou substitué une, deux ou trois fois avec -OH, un halogène, -NO₂ et/ou -NH₂,
les groupes cités ci-dessus aux points 8) à 11) étant non substitués ou substitués indépendamment l'un de l'autre, une, deux ou trois fois avec -OH, un halogène, -NO₂ et/ou -NH₂,
b) les composés céto étant lie 4-chloroacétoacétate d'éthyle, l'acétoacétate de méthyle, la 4-hydroxy-2-butanone, le pyruvate d'éthyle, le glyoxylate d'éthylphényle, la 1,4-dichloro-2-butanone, le 4-bromoacétoacétate d'éthyle, la 1,1-dichloroacétone, la 1,1,3-trichloroacétone, la 1,1,1-trifluoroacétone, la 1-chloroacétone ou la 2,5-hexanedione,
**caractérisé en ce que**
un mélange liquide biphasique qui contient
(a) au moins 5 % en poids/volume d'un des composés céto cités plus haut,
(b) au moins 10 % zen volume de 4-méthyl-2-pentanol, de 5-méthyl-2-hexanol et/ou de 2-heptanol et
(c) de l'eau,
est traité avec une oxydoréductase en présence de NAD (P) H comme co-facteur pour, dans le cas (a), former un composé chiral hydroxyle de formule générale II
R₁-CH(OH)-R₂ (II)
dans laquelle R₁ et R₂ ont les sens donnés plus haut, et dans le cas (b), former un composé chiral hydroxyle correspondant.

2. Procédé selon la revendication 1, **caractérisé en ce que** l'oxydoréductase et d'origine microbienne.

3. Procédé selon la revendication 1, **caractérisé en ce que** l'oxydoréductase provient de bactéries du groupe lactobacillales ou de levures.

4. Procédé selon la revendication 3, **caractérisé en ce que** l'oxydoréductase provient de bactéries du genre *Lactobacillus* ou de levures des genres *Pichia*, *Candida, Pachysolen, Debaromyces* ou *Issatschenkia.*

5. Procédé selon l'une des revendications 1 à 4, **caractérisé en ce que** le mélange liquide biphasique contient lors de l'utilisation d'une oxydoréductase d'origine microbienne au moins 40 % en volume de 4-méthyl-2-pentanol, le 5-méthyl-2-hexanol et/ou de 2-heptanol.

6. Procédé selon la revendication 5, **caractérisé en ce que** le mélange de liquide biphasique contient entre 40 et 80 % en volume de 4-méthyl-2-pentanol, de 5-méthyl-2-hexanol et/ou de 2-heptanol.

7. Procédé selon l'une des revendications 1 à 6, **caractérisé en ce que** le mélange liquide biphasique contient de 2 à 50 % en poids/volume d'un des composés céto cités plus haut.

8. Procédé selon la revendication 7, **caractérisé en ce que** le mélange de liquide biphasique contient entre 10 et 50 % en poids/volume de l'un des composés céto cités plus haut.
